# EUROPEAN PATENT APPLICATION

(11) **EP 4 821 502 A1**
(43) Date of publication of application: **14.10.2026**
(21) Application number: 24899789.2
(22) Date of filing: 03.12.2024
(51) Int. Cl.: A61K 38/00, A61K 38/20, C07K 14/54, C12N 7/01, A61P 35/00

(54) **USE OF DR-18 AND HERPES SIMPLEX VIRUS IN PREPARATION OF ANTITUMOR DRUG**

(30) Priority: 04.12.2023 CN 202311650826
(71) Applicant: Guangzhou Virotech Pharmaceutical Co., Ltd., Guangzhou, Guangdong 510000 (CN)
(72) Inventor: LIN, Ziqing, Guangzhou, Guangdong 510000 (CN); WU, Guang'en, Guangzhou, Guangdong 510000 (CN); YUAN, Huiyan, Guangzhou, Guangdong 510000 (CN); LING, Xiaohui, Guangzhou, Guangdong 510000 (CN); LIU, Yichun, Guangzhou, Guangdong 510000 (CN)
(74) Representative: J A Kemp LLP
(86) International application number: PCT/CN2024/136267
(87) International publication number: WO 2025/119151

(57) **Abstract**

The present disclosure belongs to the field of biomedicine, and relates to the use of DR-18 and herpes simplex virus in preparation of antitumor drug. The present disclosure finds for the first time that herpes simplex virus has a synergistic anti-tumor effect in combination with DR-18. The disclosure also relates to an anti-tumor pharmaceutical composition containing the herpes simplex virus and DR-18, and use of the herpes simplex virus and DR-18 in the preparation of an anti-tumor drug.

## Description

### FIELD

The present disclosure belongs to the field of biomedicine, and relates to the use of DR-18 and herpes simplex virus in preparation of antitumor drug.

### BACKGROUND

Malignant tumors are the first fatal disease threatening human health. According to the data of *Global Cancer Statistics Report*, there were 19.29 million new cancer cases worldwide and 9.95 million cancer-related deaths in 2020(SUNG H, FERLAY J, SIEGEL R L, et al.Global cancer statistics 2020: GLOBOCAN estimates of incidence and mortality worldwide for 36 cancers in 185 countries[J].CA Cancer J Clin, 2021,0:1-41.). It is estimated that the number of new global cancer cases will reach 28.4 million by 2040, an increase of 47% compared with 2020. The increase in cases will be the largest in less developed and moderately developed countries, reaching 95% and 64% respectively (Cao Maomao, Chen Wanqing. Interpretation of GLOBOCAN 2020 Global Cancer Statistical Data[J]. Chinese Journal of Medical Frontiers (Electronic Edition), 2021,13(3):63-69.). Therefore, there is an extensive demand for efficient and economical anti-tumor drugs among the general public (Wanqing Chen, Rongshou Zheng, et al. Cancer Statistics in China, 2015[J]. CA Cancer J Clin,2016,6(6):115-132.).

The traditional treatment methods for malignant tumors comprise operations, radiotherapy and chemotherapy, which have made great progress in the past decades, but still cannot substantially improve the long-term survival rate of patients, especially patients with refractory tumors such as liver cancer, glioma, pancreatic cancer and osteosarcoma. Cytotoxic drugs and molecular targeted drugs are currently used as first-line clinical anti-tumor drugs and have the limitations of great toxic and side effects and/or easy generation of drug resistance. Therefore, a novel treatment means and a novel therapeutic drug with high efficiency and low toxicity are urgently needed for the treatment of malignant tumors.

Currently, immunotherapy is a cancer treatment of great interest. The immunotherapy is different from the direct action of the traditional operation, radiotherapy, chemotherapy and targeted drugs, kills the tumor by activating the immune system of the patient, and has small influence on normal tissues. Immunotherapy remains effective against some advanced tumors, and can even prevent tumor recurrence to achieve thorough cure. The classes of clinically used drugs currently approved by the U.S. Food and Drug Administration (FDA) for cancer immunotherapy comprise: 1. immune checkpoint inhibitors, such as PD-1 antibody (Nivolumab, Pembrolizumab, etc.), PD-L1 antibody (Atozolizumab), and CTLA4 antibody (Iplimumab); 2. a plurality of monoclonal antibodies aiming at surface tumor-associated antigens, such as the monoclonal antibody rituximab of anti-CD 20 and the like; 3. two immunostimulatory cytokines (interferon IFN- α) and interleukin 2 (IL-2); 4. immunogenic cell death inducers such as cyclophosphamide, oxaliplatin and the like; 5. adoptive chimeric antigen receptor T cell therapy (CART); 5. bacillus Calmette Guerin (BCG); 6. dendritic cell-based cancer vaccines; 7. oncolytic Viruses (OV), and the like.

Oncolytic virus therapy is a novel anti-tumor immunotherapy that integrates targeted therapy, immunotherapy and gene therapy. It can selectively infect and directly kill tumor cells, and further activate anti-tumor immune responses by exposing tumor/viral antigens and releasing cytokines, thereby exerting tumor-suppressive effects directly or indirectly. In addition, oncolytic viruses themselves can serve as vectors carrying suicide genes, immunomodulatory genes, pro-apoptotic genes, anti-angiogenic genes and other genes to further regulate the tumor microenvironment and enhance anti-tumor efficacy. Compared with conventional clinical treatment methods currently applied, oncolytic virus therapy possesses the advantages of potent killing effect, high safety and low cost.

With unique multiple anti-tumor pathways, numerous team scholars are working to develop this therapy, however only four oncolytic virus products have been approved on the market to date for clinical treatment of tumors, including RIGVIR, marketed in Latvia in 2003, Ancheri (H101), marketed in China in 2005, IMLYGIC (T-Vec), marketed in the United states in 2015, and Delytact, marketed in Japan in 2021.

In addition, a variety of oncolytic viruses are in preclinical research, ranging from the original natural viruses to viruses subjected to gene editing, and the kinds of oncolytic viruses have also been developed from the original herpes viruses to more than ten commonly used viruses, of which adenovirus has become the most commonly used oncolytic virus due to its ease of gene editing and flexibility of use. Other commonly used viruses include herpes simplex virus, vaccinia virus, newcastle disease virus, measles virus, reovirus, coxsackievirus, poliovirus, and the like.

IL-18 is a cytokine that activates immunity, stimulates T cells, NK cells and bone marrow cells, has the ability to activate anti-tumor immune cells, and thus is a candidate molecule for the treatment of cancer. However, IL-18BP (IL-18 binding protein) produced in the tumor microenvironment can act as a secreted immune checkpoint molecule, competitively binding to IL-18 and blocking its binding to receptors, limiting the effectiveness of IL-18 immunotherapy, rendering immune cells unable to activate anti-tumor immune responses. DR18 (modified IL-18) was obtained by site-directed mutagenesis of key amino acids of the IL-18BP binding site. DR18 can effectively avoid binding with IL-18BP, and further effectively bind with IL-18 receptor, and play a role in activating the anti-tumor immune response. By utilizing the characteristic that oncolytic virus is specifically replicated in tumor, DR18 with remarkable immune activation effect can be specifically and highly and intensively expressed in the tumor part, so that the concentration of the DR18 in the tumor is obviously higher than that of other normal tissues, thereby improving the curative effect and reducing the potential risk brought by systemic exposure.

### SUMMARY

In one aspect, the present disclosure provides the use of DR-18 in the preparation of an anti-tumor drug for use in combination with herpes simplex virus.

In one aspect, the present disclosure provides the use of herpes simplex virus in the preparation of an anti-tumor drug for use in combination with DR-18.

In one aspect, the present disclosure provides the use of DR-18 in the preparation of an anti-tumor synergist or an agent for reversing drug resistance for herpes simplex virus. In another aspect, the disclosure provides the use of herpes simplex virus in the preparation of DR-18 anti-tumor synergist or an agent for reversing drug resistance.

A drug resistance reversal agent means that when certain oncolytic viruses are used as an anti-tumor drug for the treatment of tumors, some tumors are not very sensitive to oncolytic viruses, or such tumors are resistant to oncolytic viruses. In this case, oncolytic viruses can be used in combination with DR-18 (acting as an agent for reversing drug resistance) so as to reverse the resistance of tumors to the oncolytic viruses; or conversely, when some anti-tumor substances are used for the treatment of tumors, some tumors are not very sensitive to the drugs, or such tumors are resistant to the substances. In this case, oncolytic viruses can be used as a drug resistance reversal agent in combination with these substances so as to reverse the resistance of tumors to the substances.

DR-18, a class of mutated IL-18 that binds to and activates IL-18 receptors and their downstream pathways, and does not bind IL-18BP. For example, U.S. patent publication nos.: 2019/0070262; and Zhou et al, Nature (2020)583:609-614, disclose some human DR-18, and some murine DR-18.

In some embodiments, the DR-18 is a human DR-18; in some embodiments, the human DR-18 comprises at least one mutation relative to wild-type human IL-18.

In some embodiments, the human DR-18 comprises one or more of the following mutations relative to wild-type human IL-18: M51K, K53S, Q56L, P57A, M60L, S105D, D110S and N111R.

In some embodiments, the human DR-18 comprises the following mutations relative to wild-type human IL-18: M51K, K53S, Q56L, P57A, M60L, S105D, D110S and N111R.

In some embodiments, the human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 3.

In some embodiments, the amino acid sequence of human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence set forth in SEQ ID NO: 1; in some embodiments, the amino acid sequence of the wild-type human IL-18 is shown as SEQ ID NO: 3.

In some embodiments, the DR-18 is murine DR-18; in some embodiments, the murine DR-18 comprises at least one mutation relative to a wild-type murine IL-18.

In some embodiments, the murine DR-18 comprises one or more of the following mutations relative to a wild-type murine IL-18: N1H, M50A, K52G, E55R, V56A, and L59K; in some embodiments, the murine DR-18 comprises the following mutations relative to a wild-type murine IL-18: N1H, M50A, K52G, E55R, V56A, and L59K.

In some embodiments, the murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 4. In some embodiments, the amino acid sequence of murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence set forth in SEQ ID NO: 2; in some embodiments, the amino acid sequence of the wild-type murine IL-18 is shown as SEQ ID NO: 4.

In some embodiments, the herpes simplex virus is herpes simplex virus type I and/or herpes simplex virus type II; in some embodiments, the herpes simplex virus is selected from wild-type virus strains or naturally attenuated strains, genetically engineered selectively attenuated strains, gene-loaded virus strains, and gene transcription-targeted virus strains. An example of such a genetically engineered selectively attenuated strain is G207, where the gene γ34.5 has been deleted from G207 and this gene is the neurotoxicity determinant of HSV-1.

As used in this disclosure, "human DR-18" and "DR 18" may be used interchangeably; the terms "murine DR-18" and "mDR 18" are used interchangeably.

As used in this disclosure, "mutation", "mutant," or "variant" refers to an alteration of a nucleic acid or polypeptide sequence relative to a reference sequence (which may be a naturally occurring normal or "wild-type" sequence), and includes translocations, deletions, insertions, and substitutions/point mutations. "mutant" or "variant" as used herein refers to a nucleic acid or protein that comprises a mutation.

As used in this disclosure, the term "wild-type" refers to a gene or gene product isolated from a natural source. A wild-type gene is that which is most frequently observed in a population and is thus arbitrarily designated the "normal" or "wild-type" form of the gene. Conversely, the terms "modified", "variant" or "mutant" refer to a gene or gene product that has a modification in sequence and/or functional properties (i.e., altered properties) as compared to the wild-type gene or gene product.

In one aspect, the present disclosure provides a technical solution for the treatment of tumors by combining DR-18 with herpes simplex virus. In some embodiments, the term "combination" is to be interpreted broadly, e.g., pharmaceutical composition, pharmaceutical kit.

As used in this disclosure, the term "pharmaceutical combination" means a product resulting from the mixing or combining of more than one active ingredient, including fixed as well as non-fixed combinations of active ingredients. The terms "coadministration" or "combined administration" and the like as used herein are intended to encompass the administration of a selected therapeutic agent to a single patient, and are intended to encompass treatment regimens in which the drugs are not necessarily administered by the same route of administration or not necessarily at the same time.

As used in this disclosure, the term "fixed combination" means that the active ingredients (e.g., herpes simplex virus and DR-18) are administered to a patient simultaneously in a single entity or dosage form.

As used in this disclosure, the term "non-fixed combination" means that the active ingredients (e.g., herpes simplex virus and DR-18) are administered to a patient as separate entities either simultaneously, concurrently or sequentially with no specific time limit, wherein such administration provides therapeutically effective levels of both compounds in vivo, preferably at the same time. For example, a non-fixed combination may be two capsules each containing one active ingredient for the purpose of bringing the two active ingredients together in vivo to treat a patient.

As used in this disclosure, "in combination" or "used in combination" means that two or more active substances may be administered to a subject together in a mixture, each simultaneously as a single formulation, or each sequentially in any order (e.g., simultaneously, sequentially, intermittently) as a single formulation.

As used in this disclosure, the term "in combination with......," when referring to administration of multiple agents to a subject, refers to administration of a first agent to the subject with at least one other (i.e., second, third, fourth, fifth, etc.) agent. As used in this disclosure, an agent (e.g., DR-18) is considered to be administered in combination with a second agent (e.g., herpes simplex virus) if, upon administration of the second agent, the biological effect resulting from administration of the first agent persists in the subject such that the therapeutic effects of the first and second agents overlap. Administration of a first agent (e.g., DR-18) provides a therapeutic effect over an extended period of time, and administration of a second agent (e.g., herpes simplex virus) provides its therapeutic effect while the therapeutic effect of the first agent is still ongoing, and thus the second agent is considered to be administered in combination with the first agent, even though the first agent may be administered at a time point (e.g., days or weeks) that is remote from the time of administration of the second agent. In some embodiments, a first agent is considered to be administered in combination with a second agent if the first and second agents are administered simultaneously (within 30 minutes of each other), contemporaneously, or sequentially. In some embodiments, a first agent is considered to be administered "contemporaneously" with a second agent if the first and second agents are administered within about 24 hours of each other (e.g., within about 12 hours of each other, within about 6 hours of each other, within about 2 hours of each other, or within about 30 minutes of each other). The term "in combination with ... ..." should also be understood to apply where the first and second agents are co-formulated in a single pharmaceutically acceptable formulation and the co-formulation is administered to a subject. In some embodiments, the DR-18 and herpes simplex virus are administered or applied sequentially, e.g., one agent is administered followed by one or more other agents. In some embodiments, DR-18 and the herpes simplex virus are administered simultaneously, e.g., two or more agents are administered at or about the same time; the two or more agents may be present in two or more separate formulations, or combined into a single formulation (i.e., a co-formulation). Whether these agents are administered sequentially or simultaneously, as used in this disclosure, they are considered to be administered/used in combination.

In one aspect, the present disclosure provides the use of DR-18 in the preparation of an anti-tumor pharmaceutical combination comprising a herpes simplex virus in combination with DR-18.

In one aspect, the present disclosure provides the use of a herpes simplex virus for the preparation of an anti-tumor pharmaceutical combination comprising DR-18 in combination with a herpes simplex virus.

In one aspect, the present disclosure provides a pharmaceutical combination for the treatment of tumors, comprising:
DR-18, and
herpes simplex virus.

In some embodiments, the pharmaceutical combination is a pharmaceutical composition or a pharmaceutical kit; in some embodiments, the pharmaceutical composition comprises a mixture of DR-18 and herpes simplex virus; in some embodiments, the pharmaceutical kit comprises separately packaged DR-18 and separately packaged herpes simplex virus.

In one aspect, the present disclosure provides a pharmaceutical composition for the treatment of tumors, comprising:
DR-18, and herpes simplex virus.

In one aspect, the present disclosure provides a pharmaceutical kit comprising:
DR-18, and herpes simplex virus.

In some embodiments, the pharmaceutical kit comprises separately packaged DR-18 and separately packaged herpes simplex virus.

A pharmaceutical kit differs from the pharmaceutical composition in that DR-18 and herpes simplex virus are in separate dosage forms and independently packaged. For example, DR-18 is contained in pills, capsules, tablets or ampoules, and herpes simplex virus is contained in separate pills, capsules, tablets or ampoules. In some embodiments, the herpes simplex virus, DR-18, and combinations of herpes simplex virus and DR-18, may also contain one or more adjuvants. The adjuvant refers to an ingredient that can assist the therapeutic efficacy of drugs in a pharmaceutical composition. The pharmaceutical kit may also contain separately packaged DR-18, and separately packaged herpes simplex virus. Administration of DR-18, and the herpes simplex virus in the pharmaceutical kit, may be simultaneous or in any sequential order, such as administration of DR-18 prior to the herpes simplex virus, or administration of DR-18 after the herpes simplex virus, or both. In various embodiments, the patient may be a mammal.

In some embodiments, the composition/pharmaceutical kit further comprises a pharmaceutically acceptable carrier.

In some embodiments, the ratio of DR-18 to herpes simplex virus is about: 0.01-200 mg : 10³-10⁹ PFU; in some embodiments, the ratio of DR-18 to herpes simplex virus is from about 0.1-200 mg: 10⁴-10⁹ PFU; in some embodiments, the ratio of DR-18 to herpes simplex virus is about 0.1-100: 10⁵-10⁹ PFU.

In some embodiments, the dosage used is: the dosage range of DR-18 is about 0.01-10 mg/kg, and the titer of herpes simplex virus is about MOI 10³-10⁹ PFU/kg; in some embodiments, DR-18 is administered in a dosage range of about 0.1-5 mg/kg, and the herpes simplex virus is administered at a titer of about MOI 10⁴-10⁹ PFU/kg; in some embodiments, DR-18 is administered in a dosage range of about 0.05 to about 0.5 mg/kg, and the herpes simplex virus is administered at a titer of about MOI 10⁵-10⁹ PFU/kg.

In some embodiments, the DR-18 is administered intraperitoneally; in some embodiments, the herpes simplex virus is administered intratumorally, or intravenously.

In one aspect, the disclosure also provides the use of the pharmaceutical combination/pharmaceutical composition/pharmaceutical kit in the preparation of a drug for the treatment of tumors.

In some embodiments, the tumor is a solid tumor or a hematological tumor.

In some embodiments, the solid tumor is selected from one or more of intestinal cancer, pancreatic cancer, liver cancer, bladder cancer, breast cancer, cervical cancer, prostate cancer, glioma, melanoma, nasopharyngeal carcinoma, lung cancer, osteosarcoma, or gastric cancer.

In some embodiments, the invention provides a method of treating a tumor, comprising: administering any of the pharmaceutical combinations to a subject having a tumor.

In some embodiments, the nucleotide sequence of the human DR-18 is set forth in SEQ ID NO: 5.

In some embodiments, the nucleotide sequence of the murine DR-18 is as set forth in SEQ ID NO: 6.

In some embodiments, unexpectedly distinct effects were observed in the combination of some oncolytic viruses with DR-18, some of which were found to exhibit significant mutual antagonism or inhibition, yet in contrast to these phenomena, the combination of herpes simplex virus with DR-18 exhibited significant synergistic effects in the testing of a variety of tumors, which was an unexpected discovery with promising development prospects.

### BRIEF DESCRIPTION OF THE DRAWINGS

Fig. 1 shows the effect of inactivated supernatants of different oncolytic viruses infecting different tumor cells in example 1 on DR18 activity, wherein * indicates P < 0.05, ** indicates P < 0.01, and *** indicates P < 0.001.
Fig. 2 shows the effect of each OVs-SN on DR18 activity in example 1, wherein * indicates P < 0.05, ** indicates P < 0.01, and *** indicates P < 0.001, and n.s. indicates that the difference is not statistically significant.
Fig. 3 shows a curve of the change in body weight of the animals of example 2, with n.s. indicating that the difference is not statistically significant.
Fig 4 shows a tumor volume growth curve of the group treated with the combination of HSV and mDR18 in example 2, wherein * indicates P < 0.05, ** indicates P < 0.01, and *** indicates P < 0.001, and n.s. indicates that the difference is not statistically significant.
Fig. 5 shows a tumor volume growth curve of the group treated with ADV and mDR18 in example 2, wherein * indicates P < 0.05, ** indicates P < 0.01, and *** indicates P < 0.001, and n.s. indicates that the difference is not statistically significant.
Fig. 6 shows a tumor volume growth curve of the group treated with VSV and mDR18 in example 2, wherein * *indicates P < 0.05,* ** indicates P < 0.01, and *** indicates P < 0.001, and n.s. indicates that the difference is not statistically significant.
Fig. 7 shows an animal body weight change curve in example 3, wherein n.s. indicates that the difference is not statistically significant.
Fig. 8 shows a tumor volume growth curve of the group treated with HSV and mDR18 in example 3, wherein * *indicates P < 0.05,* ** indicates P < 0.01, and *** indicates P < 0.001, and n.s. indicates that the difference is not statistically significant.
Fig. 9 shows a tumor volume growth curve of the group treated with ADV and mDR18 in example 3, wherein * *indicates P < 0.05,* ** indicates P < 0.01, and *** indicates P < 0.001, and n.s. indicates that the difference is not statistically significant.

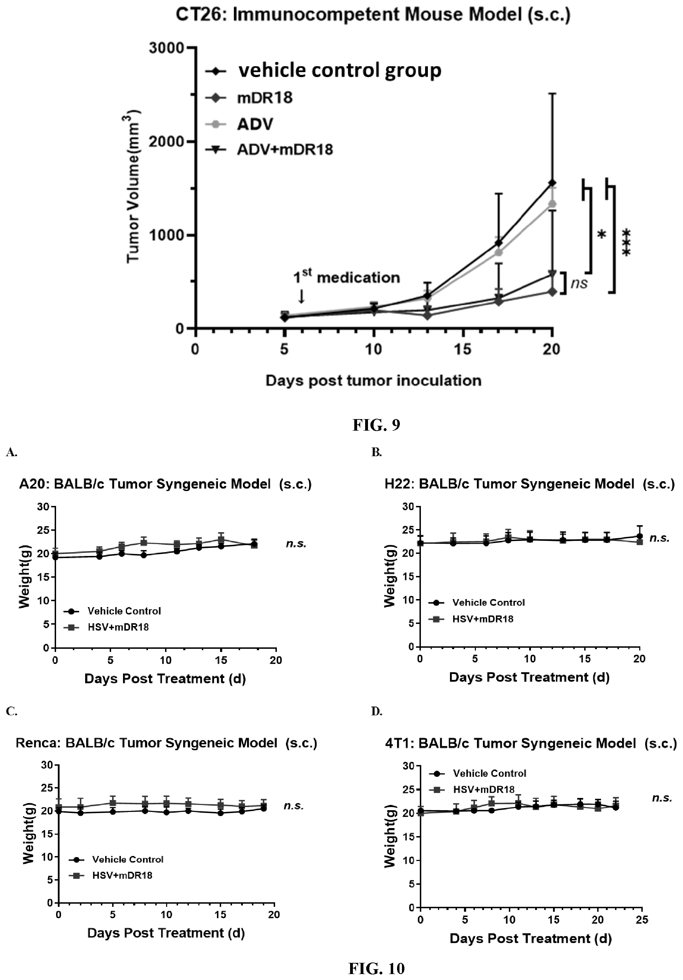

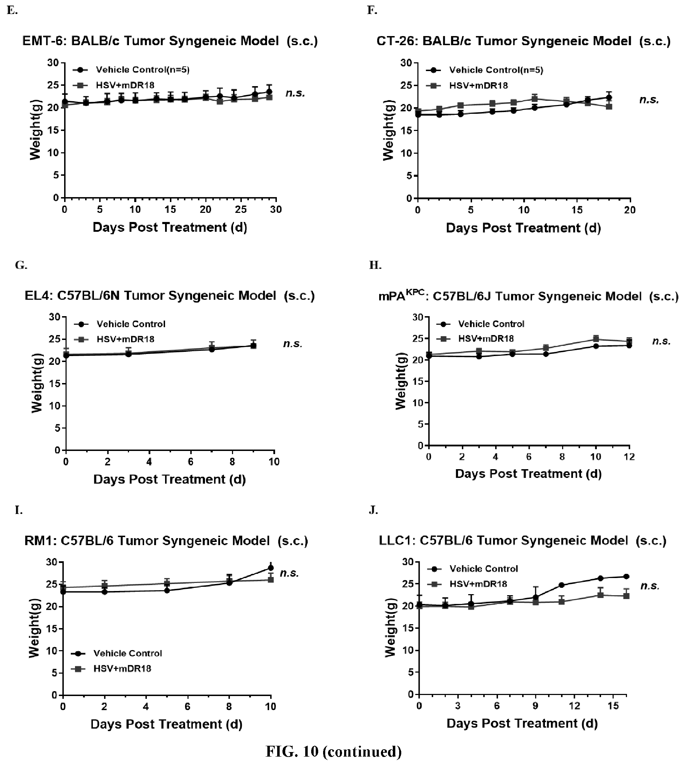

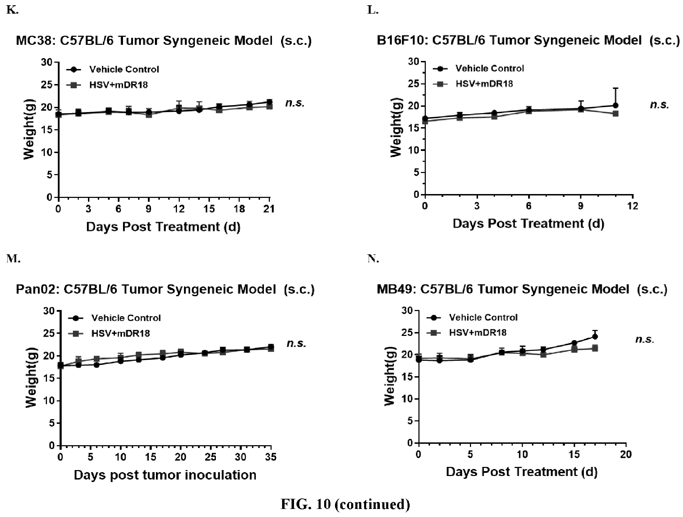

Fig. 10 shows an animal body weight change curve in example 4, wherein n.s. indicates that the difference is not statistically significant. Wherein: A. Mouse lymphoma A20 tumor-bearing model; B. Mouse hepatocellular carcinoma H22 tumor-bearing model; C. Mouse renal carcinoma Renca tumor-bearing model; D. Mouse breast cancer 4T1 tumor-bearing model; E. Mouse breast cancer EMT6 tumor-bearing model; F. Mouse colon cancer CT26.WT tumor-bearing model; G. Mouse lymphoma EL4 tumor-bearing model; H. Mouse pancreatic cancer mPAKPC. tumor-bearing model; I. Mouse prostate cancer RM-1 tumor-bearing model; J. Mouse lung cancer LLC1 tumor-bearing model; K. Mouse colon cancer MC38 tumor-bearing model; L. Mouse melanoma B16-F10 tumor-bearing model; M. Mouse pancreatic cancer Pan02 tumor-bearing model; N. Mouse bladder cancer MB49 tumor-bearing model.

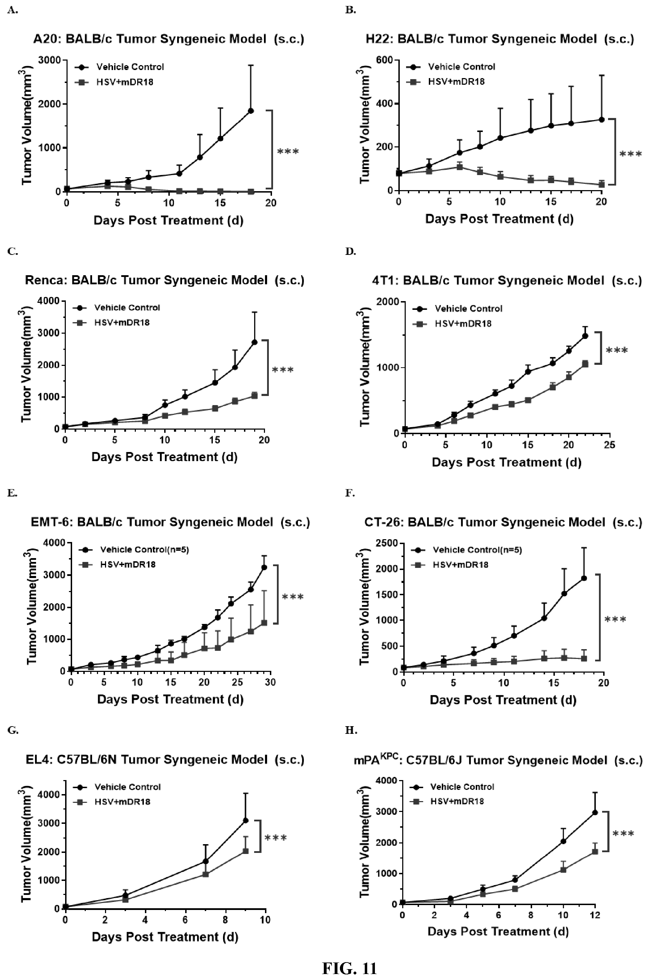

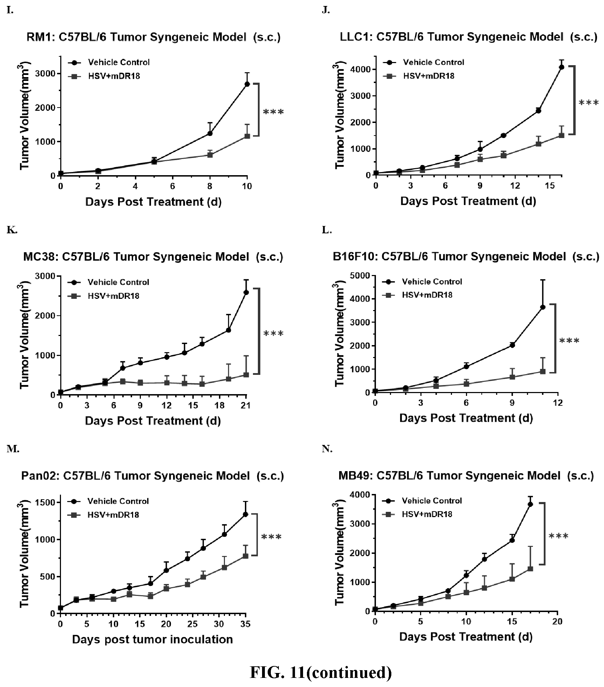

Fig. 11 shows a tumor volume growth curve in example 4, wherein * *indicates P < 0.05,* ** indicates P < 0.01, and *** indicates P < 0.001, and n.s. indicates that the difference is not statistically significant. Wherein: A. Mouse lymphoma A20 tumor-bearing model; B. Mouse hepatocellular carcinoma H22 tumor-bearing model; C. Mouse renal carcinoma Renca tumor-bearing model; D. Mouse breast cancer 4T1 tumor-bearing model; E. Mouse breast cancer EMT6 tumor-bearing model; F. Mouse colon cancer CT26.WT tumor-bearing model; G. Mouse lymphoma EL4 tumor-bearing model; H. Mouse pancreatic cancer mPAKPC. tumor-bearing model; I. Mouse prostate cancer RM-1 tumor-bearing model; J. Mouse lung cancer LLC1 tumor-bearing model; K. Mouse colon cancer MC38 tumor-bearing model; L. Mouse melanoma B16-F10 tumor-bearing model; M. Mouse pancreatic cancer Pan02 tumor-bearing model; N. Mouse bladder cancer MB49 tumor-bearing model.

### DETAILED DESCRIPTION

The technical solutions of the present invention are further illustrated below through specific examples, which shall not be construed as limiting the protection scope of the present invention. Any non-essential modifications and adjustments made by others based on the concept of the present invention shall still fall within the protection scope thereof.

The materials and experimental procedures used in the present invention are, unless otherwise specified, conventional.

### Example 1 Evaluation of the Effect of supernatants from different tumor cells infected with various oncolytic viruses on DR18 Activity Using IL-18 reporter cell model

IL-18 reporter cell model: the cell expresses IL-18 receptor, IL-18 downstream pathway molecules and luciferase, and IL18 binds to the receptor and mediates luciferase expression, the reading of which represents the activation effect of the pathway. The pathway is a key pathway for activating anti-tumor immunity in the DR18 body and can indirectly reflect the anticancer activity of DR18.

### 1. Experimental materials

1.1 Oncolytic virus: HSV: attenuated oncolytic herpes simplex virus type 1 with neurovirulence factor γ34.5 gene knock-out was purchased from Wuhan Shumi.

ADV: E1a-CR2-24bp gene and E3 region gene knock-out oncolytic adenovirus; VSV: attenuated oncolytic vesicular stomatitis virus with G gene knockout, M gene V48R & M51R mutations; REO: serotype 3 oncolytic reovirus. They are commercially available products, customized products constructed by conventional methods, or independently constructed by Guangzhou Virotech Pharmaceutical Co., Ltd. in accordance with conventional methods.

1.2 Experimental cells: IL-18 Reporter cells (H _ IL18 Reporter 293 Cell Line), prostate cancer cells (DU145), bladder cancer cells (ScaBER, J82, HT1376, 5637), pancreatic cancer cells (MIA-Paca-2, Capan2), cervical cancer cells (HCC 94), intestinal cancer cells (DLD-1), liver cancer cells (Hep 3B), lung cancer cells (HCC-827), glioma cells (LN 18, U-138 MG), osteosarcoma cells (MNNG/HOS). The cells were purchased from ATCC, Cell Bank of the Chinese Academy of Sciences, China Center for Type Culture Collection, Geniou Biotechnology, Saiku Biotechnology, Foxase Biotechnology, Tongpai Biotechnology and other suppliers.

1.3 Experimental reagents: human DR18 recombinant protein (SEQ ID NO: 1), DMEM medium, MEM medium, RPMI 1640 medium, McCoy's 5A medium, IMDM medium, EMEM medium, Fetal Bovine Serum, Puromycin, Blasticidin, G418, ONE-Glo^{™} Luciferase Assay System.

1.4 Experimental apparatus: an inverted microscope, a biological safety cabinet, a carbon dioxide incubator and an enzyme-labeling instrument.

### 2. Experimental method

2.1 Tumor cell model establishment:
   selecting proper culture conditions and passage ratio according to the specifications of each tumor cell for cell expansion passage, and inoculating the cells into a T25 cell culture flask according to 3×10⁴/cm² when the cells enter a logarithmic growth phase and the number of the cells is enough.
2.2 Oncolytic virus infection of tumor cells:
   Sixteen to twenty-four hours after tumor cell inoculation, complete cell adherence was observed under a microscope. Each type of tumor cell was infected with virus at a dosage of MOI=1 PFU/cells according to the number of seeded cells.
2.3 infection supernatant collection and inactivation:
   Forty-eight hours after tumor cell infection with oncolytic viruses, cytopathic effects were observed and photographed under a microscope. According to the characteristics of the viruses, the supernatants (SN) of virus-infected cells were inactivated by ultraviolet irradiation or filtered through 0.1 µm filters. The inactivated supernatants were collected into 4.5 mL cryotubes and stored in a refrigerator at -80 °C for later use. Non-infected supernatants were collected by the same method as controls.
2.4 Effect of infection supernatants on DR18 biological Activity:
   ① The IL-18 reporter cells were revived and subcultured. After the cells entered the logarithmic growth phase and reached a sufficient quantity, the cells were seeded into the middle wells of 96-well plates at 1.5×10⁴ cells per well with 90 µL culture medium, and 200 µL of PBS was added to each peripheral well for edge sealing.
   ② The cells were cultured in a 37 °C incubator with 5% CO₂ for 16 to 24 hours. Afterwards, the 96-well plates were taken out and complete cell adherence with good cell morphology was observed under a microscope. DR18 protein was subjected to 3-fold serial gradient dilution using the inactivated supernatants and control supernatants obtained in Step 2.3. After thorough mixing, 10 µL of the mixture was added into each well.
   ③ After addition of DR18 protein, the cells were incubated in a 37 °C incubator containing 5% CO₂ for 16 hours.
   ④ Add 100 µL of ONE-Glo^{™} substrate, incubate for 3 minutes away from light to achieve complete cell lysis. Transfer the cell lysate to a 96-well luminescence plate, and detect luciferase activity using a microplate reader.
2.5 data processing:
   According to the obtained Relative Light Unit (RLU) values of each well, the relative luminescence rate of cells in each well was calculated by the formula: % of Max = (RLUₛₐₘₚₗₑ - RLU_{Blank} ) / (RLU_{Maximum} - RLU_{Blank}) × 100%. Fitting curves were plotted with software, and the half maximal effective concentration (EC50) value of DR18 biological activity was calculated by adopting the analytical equation of [Agonist] vs. response -- Variable slope (four parameters). The EC50 values of each group were normalized, and the relative EC50 value was calculated as follows: Relative Value = EC50_{treatment group} / EC50_{control group}.
2.6 biological statistics:
   * indicates P < 0.05, ** indicates P < 0.01 and *** indicates P < 0.001, suggesting that the differences are statistically significant.

### 3. The experimental results are as follows:

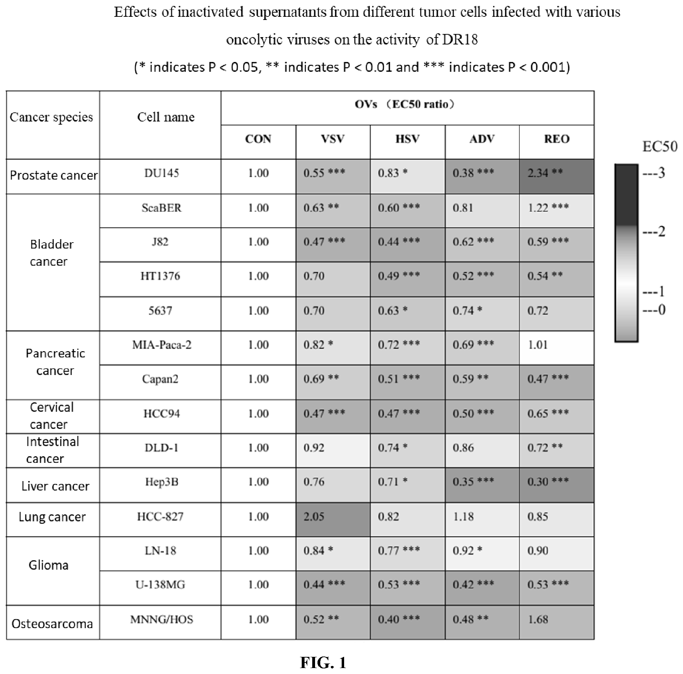

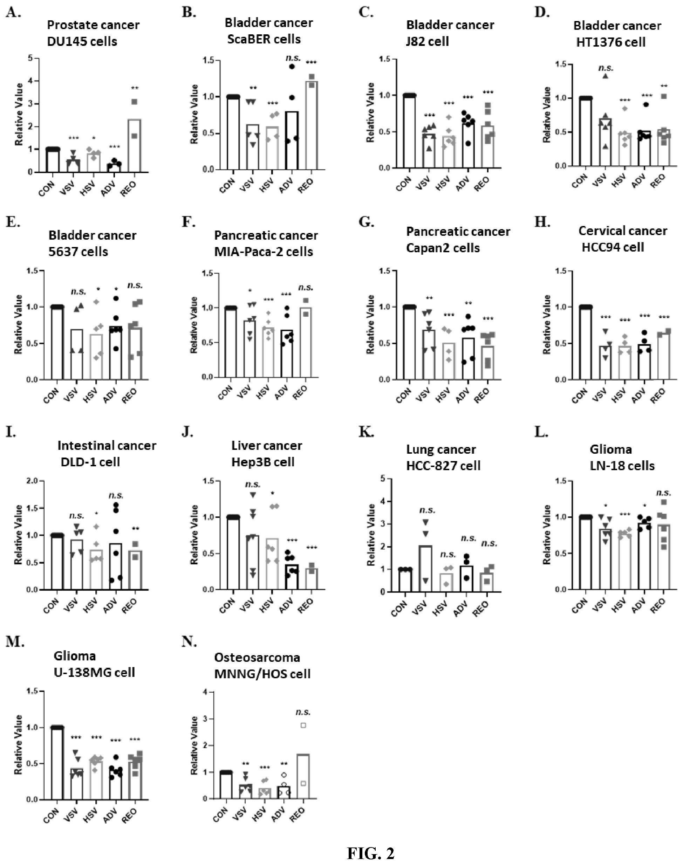

3.1 The effects of inactivated supernatants from different tumor cells infected with various oncolytic viruses on DR18 activity are shown in Figure 1. The supernatants obtained from different tumor cells infected with various oncolytic viruses were used for gradient dilution of recombinant DR18 protein, which was then applied to treat IL-18 reporter cells for 16 hours. The luciferase luminescence units were detected by instrument. The fitted curves were plotted and the EC50 values of each treatment group were calculated as shown in Figure 2, and the statistical analysis results are shown in Figure 1.

As can be seen, compared to the control group (CON) not infected with virus: the biological activity of DR18 can be remarkably promoted by infection of prostate cancer cells (DU145), bladder cancer cells (ScaBER, J82, HT1376 and 5637), pancreatic cancer cells (MIA-Paca-2 and Capan2), cervical cancer cells (HCC 94), intestinal cancer cells (DLD-1), liver cancer cells (Hep 3B), glioma cells (LN 18 and U-138 MG) and osteosarcoma cells (MNNG/HOS) by oncolytic Herpes Simplex Virus (HSV).

The biological activity of DR18 can be remarkably promoted by infection of prostate cancer cells (DU145), bladder cancer cells (ScaBER and J82), pancreatic cancer cells (MIA-Paca-2 and Capan2), cervical cancer cells (HCC94), glioma cells (LN18 and U-138MG) and osteosarcoma cells (MNNG/HOS) by oncolytic Vesicular Stomatitis Virus (VSV).

The biological activity of DR18 can be remarkably promoted by infection of prostate cancer cells (DU145), bladder cancer cells (J82, HT1376 and 5637), pancreatic cancer cells (MIA-Paca-2 and Capan2), cervical cancer cells (HCC94), liver cancer cells (Hep3B), glioma cells (LN18 and U-138MG) and osteosarcoma cells (MNNG/HOS) by oncolytic Adenovirus (ADV).

The biological activity of DR18 can be remarkably promoted by infection of bladder cancer cells (J82, HT1376), pancreatic cancer cells (Capan2), cervical cancer cells (HCC94), intestinal cancer cells (DLD-1), liver cancer cells (Hep3B) and glioma cells (U-138MG) by oncolytic Reovirus (REO). Conversely, it inhibits the biological activity of DR18 in prostate cancer cells (DU145) and bladder cancer cells (ScaBER).

The experimental result shows that the supernatants of different tumor cell models infected by each oncolytic virus have differential effect on DR18 biological activity, wherein the synergistic effect of HSV is stronger than that of other oncolytic viruses.

### Example 2 mDR18 safety and efficacy Studies in combination with various oncolytic viruses on an immuno-competent murine liver cancer model

### 1. Experimental materials

1.1 Oncolytic virus: HSV: attenuated oncolytic herpes simplex virus type 1 with neurovirulence factor γ34.5 gene knockout; ADV: E1a-CR2-24bp gene and E3 region gene knock-out oncolytic adenovirus; VSV: an oncolytic vesicular stomatitis virus attenuated by G gene knock-out and M gene V48R & M51R mutation. Viruses were purchased from Wuhan Shumi, Fubaiao biology, etc.

1.2 Recombinant protein: a modified recombinant protein (SEQ ID NO: 2) of mouse-derived interleukin-18 (mDR 18).

1.3 Tumor cells: mouse hepatoma cell line H22, cells were purchased from the China Center for Type Culture Collection.

1.4 Experimental animals: female BALB/c mice 5-7 weeks old.

### 2. Experimental method

### 2.1 Tumor-bearing mouse model establishment:

Tumor cells were revived and subcultured. After the cells entered the logarithmic growth phase and reached sufficient quantity, the cells were collected, counted and prepared into cell suspension. H22 cells were inoculated subcutaneously into the posterior dorsum of Balb/c mice at 2×10⁶cells/mouse.

### 2.2 Group administration:

After tumorigenesis, randomly grouping mice with tumor volume range of 100±40mm³, and setting 8 groups, namely a vehicle control group, an mDR18 single-use group, an HSV + mDR18 combination group, an ADV single-use group, an ADV + mDR18 combination group, a VSV single-use group and a VSV + mDR18 combination group, wherein each group comprises 5 animals; mDR18 is administered intraperitoneally with administration dosage of 0.32 mg/kg, and the administration cycle is 2 times per week and 5 times in total; each oncolytic virus was administered at a dose of 2×10⁶ PFU/mouse by intratumoral injection for 1 total administration. The day of tumor implantation was designated as D0, and observation was continued for 21 days after the administration.

### 2.3 mouse body weight and tumor body measurement:

The body weight and tumor growth of the mice were recorded every 3-4 days during the tumor formation and administration period.

### 2.4 Data processing:

Data were counted and tumor volume growth curves were plotted for each group of mice.

The Tumor Volume (TV) is calculated as: V = 1/2×a×b², wherein a and b denote a tumor body long diameter and a wide diameter, respectively.

### 2.5 Statistical analysis:

* indicates P < 0.05, ** indicates P < 0.01 and *** indicates P < 0.001, suggesting that the differences are statistically significant.

### 3. The experimental results are as follows:

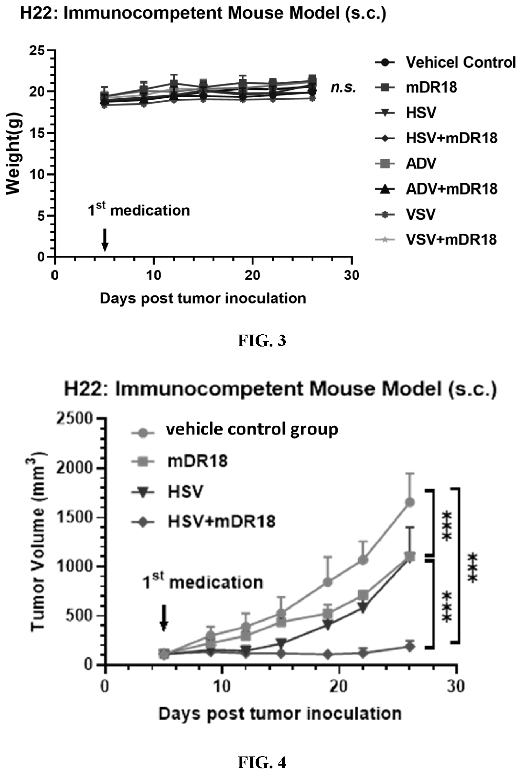

The body weights of animals in each group during the experiment are shown in Figure 3, the body weights of the animals in each group keep a steady rising trend during the experiment, and compared with an vehicle control group, the single-use or combined-use groups of the drugs have no obvious difference, which indicates that 0.32 mg/kg mDR18 is administered intraperitoneally for 2 times every week or each oncolytic virus with 2×10⁶ PFU/mouse does not cause obvious drug-related side effects in an immunocompetent murine model.

The efficacy was evaluated by plotting the change curve of tumor volume according to the tumor volume growth record of the animals as shown in FIG. 4. The results showed that compared with the vehicle control group, single use of mDR18 (P < 0.001) and combined use with HSV(P < 0.001) could significantly inhibit tumor growth in mouse hepatocellular carcinoma tumor-bearing models; compared with the single mDR18 group, there was statistically significant difference in tumor inhibitory effect in the combined use group of HSV and mDR18, indicating that the combination of HSV and mDR18 has synergistic effect.

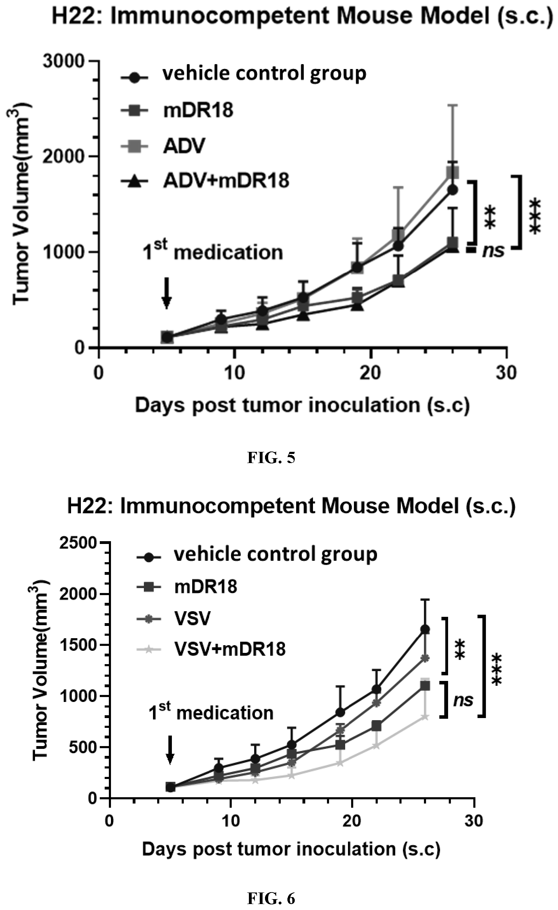

The efficacy was evaluated by plotting the change curve of tumor volume according to the tumor volume growth record of the animals as shown in FIG. 5. The results showed that compared with the vehicle control group, single use of mDR18 (P < 0.01) and combined use with ADV (P < 0.001) could significantly inhibit tumor growth in mouse hepatocellular carcinoma tumor-bearing models; compared with the single mDR18 group, there was no statistically significant difference in tumor inhibitory effect in the combined use group of ADV and mDR18, indicating that the combination of ADV and mDR18 has no synergistic effect.

The efficacy was evaluated by plotting the change curve of tumor volume according to the tumor volume growth record of the animals as shown in FIG. 6. The results show that compared with the vehicle control group, mDR18 can significantly inhibit the tumor growth of the mouse liver cancer tumor-bearing model when used alone (P < 0.01) or combined with VSV (P < 0.001); compared with the mDR18 single group, the difference of the tumor inhibition effect of the VSV and mDR18 combined group has no statistical significance, which indicates that the VSV and mDR18 combined group have no synergistic effect.

### Example 3 mDR18 safety and efficacy Studies in combination with various oncolytic viruses on immunocompetent murine intestinal cancer models

### 1. Experimental materials

1.1 Oncolytic virus: HSV: attenuated oncolytic herpes simplex virus type 1 with neurovirulence factor γ34.5 gene knockout; ADV: e1a-CR2-24bp gene and E3 region gene knock-out oncolytic adenovirus.

1.2 Recombinant protein: a modified recombinant protein of mouse-derived interleukin-18 (mDR 18).

1.3 Tumor cells: Mouse intestinal cancer cell line CT26, which was purchased from Guangzhou Jiniou Biotechnology Co., Ltd.

1.4 Experimental animals: female BALB/c mice 5-7 weeks old.

### 2. Experimental method

### 2.1 Tumor-bearing mouse model establishment:

Tumor cells were revived and subcultured. After the cells entered the logarithmic growth phase and reached sufficient quantity, they were collected, counted and prepared into cell suspension. CT26 cells were inoculated subcutaneously into the posterior dorsum of Balb/c mice at a dose of 2×10⁶ cells/mouse.

### 2.2 Group administration:

After tumorigenesis, randomly grouping mice with tumor volume range of 100±40mm³, and setting 8 groups, namely a vehicle control group, an mDR18 single-use group, an HSV single-use group, an HSV+mDR18 combination group, an ADV single-use group and an ADV+mDR18 combination group, wherein each group comprises 4 animals; the administration mode of mDR18 is intraperitoneal injection with administration dosage of 0.32 mg/kg, and the administration cycle is 2 times per week and 5 times in total; each oncolytic virus was administered at a dose of 2×10⁶ PFU/mouse by intratumoral injection for 1 total administration. The day of tumor implantation was designated as D0, and observation was continued for 14 days after the administration.

2.3 Mouse body weight and tumor body measurement:
the body weight and tumor growth of the mice were recorded every 3-4 days during the tumor formation and administration period.

### 2.4 Data processing:

Data were counted and tumor volume growth curves were plotted for each group of mice.

The Tumor Volume (TV) is calculated as: V = 1/2×a×b², wherein a and b denote a tumor body long diameter and a wide diameter, respectively.

### 2.5 Statistical analysis:

* indicates P < 0.05, ** indicates P < 0.01 and *** indicates P < 0.001, suggesting that the differences are statistically significant.

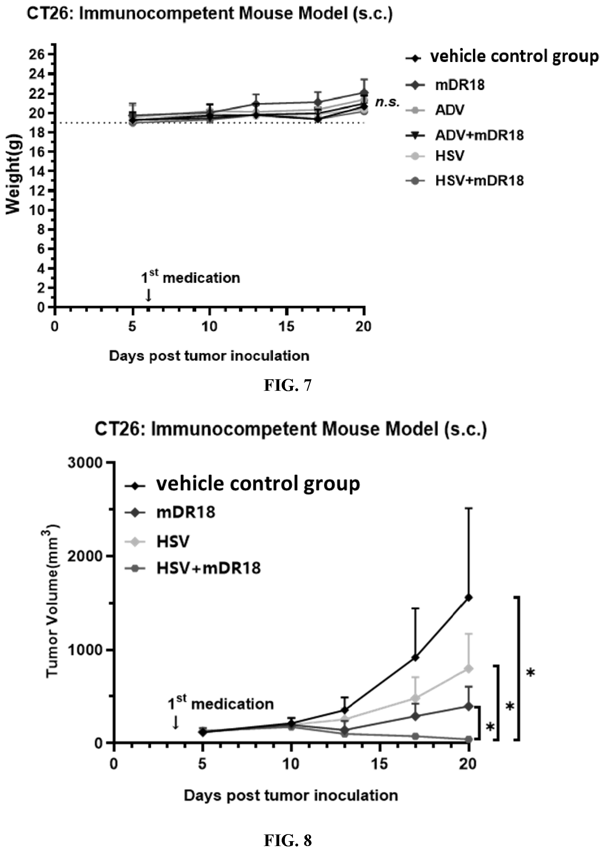

3. The experimental results are as follows:
the body weights of animals in each group during the experiment are shown in Figure 7. The body weight of mice in all groups presented a steady upward trend throughout the experiment. No significant differences were observed between each single or combined drug group and the vehicle control group. The results indicated that intraperitoneal injection of mDR18 at 0.32 mg/kg twice a week or single intratumoral injection of oncolytic viruses at 2×10⁶ PFU/mouse did not induce obvious drug-related side effects in an immunocompetent murine model.

The efficacy was evaluated by plotting the change curve of tumor volume according to the tumor volume growth record of the animals as shown in FIG. 8. The results showed that compared with the vehicle control group, combined use of HSV and mDR18 (P < 0.05) could significantly inhibit tumor growth in mouse hepatocellular carcinoma tumor-bearing models; compared with the single HSV group, combined use of HSV and mDR18 (P < 0.05) could significantly enhance the tumor inhibitory effect on mouse hepatocellular carcinoma tumor-bearing models; compared with the single mDR18 group, combined use of HSV and mDR18 (P < 0.05) could also significantly enhance the tumor inhibitory effect on mouse hepatocellular carcinoma tumor-bearing models, indicating that the combination of HSV and mDR18 has a synergistic effect.

The efficacy was evaluated by plotting the change curve of tumor volume according to the tumor volume growth record of the animals as shown in FIG. 9. The results showed that compared with the vehicle control group, single use of mDR18 (P < 0.001) and combined use with ADV (P < 0.05) could significantly inhibit tumor growth in mouse hepatocellular carcinoma tumor-bearing models; compared with the single mDR18 group, there was no statistically significant difference in the tumor inhibitory effect of combined use of ADV and mDR18, indicating that the combination of ADV and mDR18 has no synergistic effect.

### Example 4 Study on Safety and Efficacy of Combined Use of mDR18 and HSV in Tumor-bearing Models of Immunocompetent Mice

### 1. Experimental materials

1.1 Oncolytic virus: HSV: attenuated oncolytic herpes simplex virus type 1 with neurovirulence factor γ34.5 gene knockout;

1.2 Recombinant protein: a modified recombinant protein of mouse-derived interleukin-18 (mDR 18).

1.3 Tumor cells: A. Mouse lymphoma cells A20; B. Mouse hepatocellular carcinoma cells H22; C. Mouse renal carcinoma cells Renca; D. Mouse breast cancer cells 4T1; E. Mouse breast cancer cells EMT6; F. Mouse colon cancer cells CT26.WT; G. Mouse lymphoma cells EL4; H. Mouse pancreatic cancer cells mPAKPC; I. Mouse prostate cancer cells RM-1; J. Mouse lung cancer cells LLC1; K. Mouse colon cancer cells MC38; L. Mouse melanoma cells B16-F10; M. Mouse pancreatic cancer cells Pan02; N. Mouse bladder cancer cells MB49. All the above cells were derived from Guangdong Yaokang Biotechnology Co., Ltd.

1.4 Experimental animals: female BALB/c mice 6-8 weeks old; female C57BL/6 mice 6-8 week old; male C57BL/6 mice 6-8 weeks old.

### 2.1 Tumor-bearing mouse model establishment:

Tumor cells were revived and subcultured. After the cells entered the logarithmic growth phase and reached sufficient quantity, cells were harvested, counted and prepared into cell suspension: A. A20 cells were inoculated above the right dorsal thigh of BALB/c mice at 1×10⁶ cells/mouse; B. H22 cells were inoculated above the right dorsal thigh of BALB/c mice at 1×10⁶ cells/mouse; C. Renca cells were inoculated above the right dorsal thigh of BALB/c mice at 5×10⁵ cells/mouse; D. 4T1 cells were inoculated above the right dorsal thigh of BALB/c mice at 5×10⁵ cells/ mouse; E. EMT6 cells were inoculated above the right dorsal thigh of BALB/c mice at 2×10⁶ cells/mouse; F. CT26.WT cells were inoculated above the right dorsal thigh of BALB/c mice at 3×10⁵ cells/mouse; G. EL4 cells were inoculated above the right dorsal thigh of BALB/c mice at 5×10⁴ cells/mouse; H. mPAKPC cells were inoculated into the right forelimb of BALB/c mice at 5×10⁵ cells/mouse; I. RM-1 cells were inoculated above the right dorsal thigh of C57BL/6 mice at 1×10⁶ cells/mouse; J. LLC1 cells were inoculated above the right dorsal thigh of C57BL/6 mice at 3×10⁵ cells/mouse; K. MC38 cells were inoculated into the right forelimb of C57BL/6 mice at 1×10⁶ cells/mouse; L. B16-F10 cells were inoculated into the right forelimb of C57BL/6 mice at 5×10⁵ cells/mouse; M. Pan02 cells were subcutaneously inoculated into the right forelimb of C57BL/6 mice at 5×10⁶ cells/mouse; N. MB49 cells were subcutaneously inoculated into the right forelimb of C57BL/6 mice at 5×10⁶ cells/mouse.

### 2.2 Group administration:

After tumorigenesis, randomly grouping mice with tumor volume range of 60~100mm³, and setting 2 groups, namely a vehicle control group and an HSV+mDR18 group, wherein each group comprises 5 animals; the administration mode of mDR18 is intraperitoneal injection with administration dosage of 0.32 mg/kg, and the administration cycle is 2 times per week and 8 times in total; HSV was administered at a dose of 2×10⁶ PFU/mouse by intratumoral injection for 1 total administration. On the day of tumor implantation, D0, observation was continued for 1 month or ethical endpoint after dosing.

### 2.3 Mouse body weight and tumor body measurement:

The body weight and tumor growth of the mice were recorded every 3-4 days during the tumor formation and administration period.

### 2.4 data processing:

Data were counted and tumor volume growth curves were plotted for each group of mice.

The Tumor Volume (TV) is calculated as: V = 1/2×a×b², wherein a and b denote a tumor body long diameter and a wide diameter, respectively.

### 2.5 Statistical analysis:

* indicates P < 0.05, ** indicates P < 0.01 and *** indicates P < 0.001, suggesting that the differences are statistically significant.

### 3. The experimental results are as follows:

The body weights of animals in each group during the experiment are shown in fig. 10, the body weights of the animals in each group keep a steady rising trend during the experiment, and compared with a vehicle control group, the HSV and mDR18 combined group has no obvious difference, which indicates that mDR18 of 0.32 mg/kg is intraperitoneally injected 2 times a week or 2×10 ⁶ PFU/single HSV in intratumoral injection does not cause obvious medicament-related side effects in an immunocompetent murine model.

The efficacy was evaluated by plotting the change curve of tumor volume according to the tumor volume growth record of the animals as shown in FIG. 11. The results show that, when HSV is used in combination with mDR18, the growth of tumors in vivo, including homoergic mouse lymphoma cell a20 (P < 0.001), mouse liver cancer cell H22 (P < 0.001), mouse kidney cancer cell Renca (P < 0.001), mouse breast cancer cell 4T1 (P < 0.001), mouse breast cancer cell EMT6 (P < 0.001), mouse colon cancer cell CT26.WT(P < 0.001), mouse lymphoma cell EL4 (P < 0.001), mouse pancreatic cancer cell mPAKPC (P < 0.001), mouse prostate cancer cell RM-1 (P < 0.001), mouse lung cancer cell LLC1 (P < 0.001), mouse colon cancer cell MC38 (P < 0.001), mouse melanoma cell B16-F10 (P < 0.001), mouse pancreatic cancer cell Pan02 (P < 0.001), and mouse bladder cancer cell MB49 (*P* <0.001), can be significantly inhibited, compared with a vehicle control group.

The embodiments of the present disclosure are only illustrative examples, and the embodiments of the present disclosure are not limited to the above-mentioned embodiments, and any other changes, modifications, substitutions, combinations, and simplifications which do not depart from the spirit and principle of the present disclosure should be construed as equivalents and are included in the scope of the present disclosure.

## Claims

1. Use of DR-18 in the preparation of an anti-tumor drug for use in combination with herpes simplex virus.

2. Use of herpes simplex virus in the preparation of an anti-tumor drug for use in combination with DR-18.

3. The use of claim 1 or 2, wherein the DR-18 is human DR-18;
preferably, the human DR-18 comprises at least one mutation, relative to wild-type human IL-18;
preferably, the human DR-18 comprises one or more of the following mutations relative to wild-type human IL-18: M51K, K53S, Q56L, P57A, M60L, S105D, D110S and N111R;
preferably, the human DR-18 comprises the following mutations relative to wild-type human IL-18: M51K, K53S, Q56L, P57A, M60L, S105D, D110S and N111R;
preferably, the human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 3;
preferably, the amino acid sequence of human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 1;
preferably, the amino acid sequence of the wild type human IL-18 is shown as SEQ ID NO: 3.

4. The use of claim 1 or 2, wherein the DR-18 is murine DR-18;
preferably, the murine DR-18 comprises at least one mutation relative to a wild-type murine IL-18;
preferably, the murine DR-18 comprises one or more of the following mutations relative to a wild-type murine IL-18: N1H, M50A, K52G, E55R, V56A, and L59K;
preferably, the murine DR-18 comprises the following mutations relative to a wild-type murine IL-18: N1H, M50A, K52G, E55R, V56A, and L59K;
preferably, the murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 4;
preferably, the amino acid sequence of murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 2;
preferably, the amino acid sequence of the wild type murine IL-18 is shown as SEQ ID NO: 4.

5. The use of claim 1 or 2, wherein the herpes simplex virus is herpes simplex virus type I and/or herpes simplex virus type II.

6. A pharmaceutical combination for the treatment of a tumor comprising:
DR-18, and
herpes simplex virus.

7. The pharmaceutical combination of claim 6, wherein the pharmaceutical combination is a pharmaceutical composition or a pharmaceutical kit;
preferably, the pharmaceutical composition comprises a mixture of DR-18 and herpes simplex virus;
preferably, the pharmaceutical kit comprises separately packaged DR-18 and separately packaged herpes simplex virus.

8. The pharmaceutical combination of any of claims 6-7, wherein the DR-18 is human DR-18;
preferably, the human DR-18 comprises at least one mutation, relative to wild-type human IL-18;
preferably, the human DR-18 comprises one or more of the following mutations relative to wild-type human IL-18: M51K, K53S, Q56L, P57A, M60L, S105D, D110S and N111R;
preferably, the human DR-18 comprises the following mutations relative to wild-type human IL-18: M51K, K53S, Q56L, P57A, M60L, S105D, D110S and N111R;
preferably, the human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 3;
preferably, the amino acid sequence of human DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 1;
preferably, the amino acid sequence of the wild type human IL-18 is shown as SEQ ID NO: 3;
preferably, the DR-18 is murine DR-18;
preferably, the murine DR-18 comprises at least one mutation relative to a wild-type murine IL-18;
preferably, the murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 4;
preferably, the amino acid sequence of murine DR-18 comprises a sequence having at least 85% or at least 90% or at least 91% or at least 92% or at least 93% or at least 94% or at least 95% or at least 96% or at least 97% or at least 98% or at least 99%, or 100% identity to the sequence shown as SEQ ID NO: 2;
preferably, the amino acid sequence of the wild murine IL-18 is shown as SEQ ID NO: 4;
preferably, the herpes simplex virus is herpes simplex virus type I and/or herpes simplex virus type II;
preferably, the composition/pharmaceutical kit further comprises a pharmaceutically acceptable carrier.

9. The pharmaceutical combination of any of claims 6-8, wherein a ratio of the DR-18 to the herpes simplex virus is about: 0.01-200 mg:10³-10⁹ PFU; preferably about 0.1-200 mg: 10 ⁴ -10 ⁹ PFU; preferably about 0.1-100:10⁵-10⁹ PFU;
preferably, a dosages used are: a dosage range of the DR-18 is about 0.01-10 mg/kg, and a titer of the herpes simplex virus is about MOI 10³-10⁹ PFU/kg; preferably, the DR-18 is administered in a dosage range of about 0.1 to about 5 mg/kg, and the herpes simplex virus is administered at a titer of about MOI 10⁴-10⁹ PFU/kg; preferably, the DR-18 is administered in a dosage range of about 0.05 to about 0.5 mg/kg, and the herpes simplex virus is administered at a titer of about MOI 10⁵-10⁹ PFU/kg;
preferably, the DR-18 is administered intraperitoneally;
preferably, the herpes simplex virus is administered intratumorally, or intravenously.

10. The use/pharmaceutical combination of any one of claims 1-9, wherein the tumor is a solid tumor or a hematological tumor;
preferably, the solid tumor is selected from one or more of intestinal cancer, pancreatic cancer, liver cancer, bladder cancer, breast cancer, cervical cancer, prostate cancer, glioma, melanoma, nasopharyngeal carcinoma, lung cancer, osteosarcoma or gastric cancer.

11. A method for treating tumors, comprising: administering the pharmaceutical combination of any one of claims 6-9 to a subject having a tumor.
